# DEMANDE DE BREVET EUROPEEN

(11) **EP 3 461 918 A1**
(43) Date de publication de la demande: **03.04.2019**
(21) Numéro de dépôt: 18197010.4
(22) Date de dépôt: 26.09.2018
(51) Int. Cl.: C12R 1/885, C12N 1/14, C12N 9/42

(54) **SOUCHE DE TRICHODERMA REESEI HYPERPRODUCTRICE ET PRESENTANT UNE ACTIVITE BETA-GLUCOSIDASE AMELIOREE**

(30) Priorité: 29.09.2017 FR 1759144
(71) Demandeur: IFP Energies nouvelles, 92500 Rueil-Malmaison (FR)
(72) Inventeur: BIDARD-MICHELOT, Frédérique, 78620 L'ETANG LA VILLE (FR); CHAN HO TONG, Laetitia, 94360 BRY SUR MARNE (FR); MARGEOT, Antoine, 75018 PARIS (FR); COHEN, Céline, 75017 PARIS (FR)
(74) Mandataire: Cabinet Plasseraud

(57) **Abrégé**

La présente invention concerne une souche de *Trichoderma reesei* qui est hyperdroductrice et qui présente une activité β-glucosidase améliorée, ainsi que l'utilisation de ladite souche.

## Description

La présente invention concerne une souche de *Trichoderma reesei* qui est hyperdroductrice et qui présente une activité β-glucosidase améliorée, ainsi que l'utilisation de ladite souche.

*Trichoderma reesei* (*T. reesei*) est une espèce de champignon filamenteux cellulolytique, du genre *Trichoderma,* qui a été découvert pendant la Seconde Guerre mondiale dans le Pacifique Sud. Ce champignon a la capacité de sécréter une grande quantité d'enzymes cellulosiques (cellulases et hémicellulases), et il est aujourd'hui principalement utilisé dans le procédé de production des biocarburants de deuxième génération. En effet, les enzymes produites par ce champignon sont particulièrement utiles pour la transformation des matières de la biomasse végétale en bioproduits utiles à l'industrie, tels que le bioéthanol.

Les biocarburants de deuxième génération (issus de ressources non alimentaires) sont particulièrement d'intérêt de nos jours, étant donné que les biocarburants de première génération (issus de ressources alimentaires) ne peuvent être produits qu'en quantité limitée, dans la mesure où ils rentrent en concurrence avec la production alimentaire.

Le procédé de production des biocarburants de deuxième génération comprend quatre étapes principales : le prétraitement de la biomasse lignocellulosique, l'hydrolyse enzymatique de la biomasse lignocellulosique, la fermentation et la distillation.

Même si toutes les étapes de la production des biocarburants de deuxième génération peuvent et doivent être optimisées -afin d'augmenter la production-, un effort particulier est porté sur l'étape d'hydrolyse enzymatique. Cette étape d'hydrolyse met en jeu les enzymes de type cellulase produites par le champignon filamenteux *T. reesei.*

De manière plus générale, *T. reesei* peut être utilisé comme souche plateforme pour la production de protéines d'intérêt industrielles homologues ou hétérologues. Afin d'optimiser les performances de *T. reesei,* il est essentiel d'améliorer les souches de *T. reesei* produisant les protéines d'intérêt.

Parmi les méthodes d'améliorations envisagées l'ingénierie génétique de *T. reesei* est ainsi une solution. Elle permet d'améliorer les performances de sécrétion du champignon filamenteux producteur de cellulases, les propriétés des enzymes et de maitriser la stabilité des souches en conditions industrielles.

La mutagénèse est une technique communément utilisée en génie génétique. Elle vise à introduire volontairement des mutations dans l'ADN afin de créer des gènes génétiquement modifiés. Ceci peut permettre de générer des souches aux caractéristiques intéressantes d'un point de vue industriel. Il existe deux méthodes de mutagénèse couramment utilisées pour introduire des mutations chez *T. reesei* : la mutagénèse aléatoire et la mutagénèse dirigée.

La mutagénèse aléatoire consiste à induire des mutations non ciblées, n'importe où dans l'ADN. Ces mutations sont provoquées par l'exposition de l'organisme cible à des agents chimiques mutagènes ou à des radiations. Etant donné que les mutations sont un phénomène naturel, la mutagénèse aléatoire est donc considérée comme un accélérateur de ce processus naturel et les organismes ainsi obtenus sont considérés comme naturels et non comme organismes génétiquement modifiés (OGM) ; ils ne sont donc pas soumis à l'obligation de traçabilité. Cependant, cette méthode engendre en plus de la mutation responsable du caractère d'intérêt, un grand nombre de mutations indésirables dites « collatérales » qui contribuent, en s'accumulant, à l'instabilité, à la mauvaise santé, voire à la létalité de l'organisme muté.

La mutagénèse dirigée permet d'introduire des mutations identifiées dans un gène précis. Pour ce faire, l'ADN d'intérêt contenant les mutations est synthétisé puis introduit dans la cellule à muter où le mécanisme de réparation de l'ADN s'occupe de l'intégrer dans le génome. L'utilisation d'un marqueur de sélection permet d'identifier les cellules ayant intégré la mutation de celles qui ne l'ont pas intégrée. Cependant, les organismes ayant subi cette mutagénèse sont considérés comme des OGM (en raison de l'introduction d'ADN exogène), et sont donc soumis à une obligation de traçabilité.

Dans le cas de l'utilisation de *T. reesei* pour la production de biocarburants de deuxième génération, l'amélioration de l'étape d'hydrolyse, *via* l'introduction de mutations (aléatoires ou dirigées) chez *T. reesei* n'est donc pas satisfaisante, en raison de l'accumulation des mutations indésirables que cela entraine, ou bien en raison de l'introduction d'ADN exogène. Il existe donc un besoin pour une nouvelle méthode d'amélioration de l'étape d'hydrolyse.

A ce jour la reproduction sexuée de *T. reesei* n'a jamais été utilisée comme outil d'amélioration car *T. reesei* a toujours été considéré comme ne pouvant pas faire de reproduction sexuée. Néanmoins la découverte d'une sexualité chez *T. reesei* (Seidl *et al.,* 2009) a ouvert de nouvelles possibilités d'amélioration génétique des souches. La reproduction sexuée permet, entre autres, de créer de la diversité génétique, de conserver les mutations bénéfiques et de supprimer du génome les mutations « collatérales ». Les inventeurs de la présente invention ont ainsi cherché à développer les performances cellulolytiques de *T. reesei* grâce à la reproduction sexuée, notamment en croisant des souches sauvages et des souches industrielles de *T. reesei.*

La présente invention repose ainsi sur les résultats des inventeurs qui ont eu le grand mérite d'obtenir, après de nombreuses recherches, une nouvelle souche de *T. reesei* qui est hyperproductrice d'enzymes cellulolytiques. Comme indiqué dans l'exemple 1, cette souche a été obtenue par croisement et la souche hyperproductrice de cellulases RutC30 (Montenecourt and Eveleigh, 1977) avec la souche A2 (souche issue d'une ascospore isolée provenant de la souche autofertile CBS999.97). De façon surprenante, parmi les enzymes cellulolytiques produites par la souche selon l'invention, l'enzyme β-glucosidase présente une activité améliorée (en comparaison avec l'activité des parents RutC30 et A2). La souche selon l'invention est également de type sexuel *MAT1-1* et est fertile.

Dans un premier aspect, l'invention concerne ainsi une souche de *T. reesei* déposée le 03 août 2017 à la CNCM sous le numéro CNCM I-5221. Cette souche est dénommée RuA-149 dans les exemples et fait également référence à la « souche selon l'invention ». La CNCM fait référence à la Collection Nationale de Cultures de Microorganismes de l'Institut Pasteur, située 25 rue du Docteur Roux, F-75724 Paris cedex 15.

Ladite souche est avantageusement une souche hyperproductrice d'enzymes cellulolytiques, notamment une souche hyperproductrice de cellulases telles que les endoglucanases, les exoglucanases et les glucosidases. Plus particulièrement, une « souche hyperproductrice d'enzymes » selon l'invention s'entend d'une souche dont la production d'enzymes cellulolytiques, notamment les cellulases, représente entre 81% et 250% par rapport à celle de la souche RutC30 (la souche RutC30 étant la souche hyperproductrice de référence (Montenecourt and Eveleigh, 1977)). Le terme « entre 81% et 250% » s'entend de toutes les valeurs comprises entre 81 et 250, par exemple 90, 100, 110, 120, 130, 140, 150, 160, 170, 180, 190, 200, 210, 220, 230 et 240. Dans un mode de réalisation, la souche selon l'invention produit environ 30g/L de protéines, et de préférence environ 34g/L (dosages réalisés avec la méthode Lowry, voir Lowry *et al.*). La production de cellulases par une souche peut être déterminée par toutes techniques usuelles pour l'homme du métier, tel que par exemple le protocole suivant :
- la souche est cultivée en microplaque dans 2 mL de milieu inducteur de cellulases (tel que Avicel®, commercialisé par MSDS sous la référence No.: 9004-34-6).
- après sept jours de culture, la production de cellulases est mesurée par le dosage de la quantité de protéines totales sécrétées dans le surnageant selon la méthode de Bradford. Une corrélation entre protéines totales sécrétées et cellulases peut être faite car chez *T. reesei,* les exoglucanases (CBHI, CBHII) et les endoglucanases (EGI, EGII) principales peuvent représenter jusqu'à 90% de la quantité totale de protéines sécrétées (voir par exemple Markov *et al.,* 2005).

Avantageusement, ladite souche selon l'invention présente également une activité β-glucosidase améliorée. Le terme « activité β-glucosidase améliorée » s'entend d'une meilleure activité β-glucosidase par rapport à la souche RutC30 (ATCC® 56765), de préférence environ trois fois supérieure à l'activité β-glucosidase de RutC30. Selon un mode de réalisation, ladite souche présente une activité β-glucosidase d'au moins 0,53 IU/mg, et de préférence d'au moins 1,33±0,09 IU/mg. L'obtention d'une activité β-glucosidase améliorée permet d'augmenter la performance du cocktail d'enzymes (comprenant les endo/exoglucanases et les glucosidases telles que la β-glucosidase) car il s'agit de l'enzyme limitante de la dégradation de la cellulose. L'activité β-glucosidase peut être déterminée par toutes techniques usuelles pour l'homme du métier, tel que par exemple l'hydrolyse de p-nitrophenyl-β-Dglucopyranoside (pNPG) décrite ci-après :
- le dosage de l'activité β-glucosidase s'effectue sur le surnageant de culture. Les échantillons à doser sont dilués dans du tampon citrate 50 mM additionné de 0,5 g/L d'albumine sérique bovine (ou Bovine Serum Albumin) pour avoir une concentration en protéines de l'ordre de 50 à 100 mg/L ;
- 10 µL de la dilution et 90 µL de substrat pNPG préparé à 5 mM dans du tampon citrate 50 mM pH 4,8 sont ajoutés dans des tubes de 1,5 mL ;
- les tubes sont incubés 30 minutes à 50°C;
- 100 µL de Na₂C0₃ 2% sont ajoutés dans les tubes ;
- après 20 minutes d'incubation, la densité optique (absorbance) est lue à 410 nm ;
- la concentration équivalente de p-nitrophénol libérée est calculée grâce à une gamme de 25, 50, 100 et 200 µM de tampon citrate 50mM additionné de 0,5g/L de BSA.

Ladite souche selon l'invention est de type sexuel *MAT1-1.* Cette souche présente donc l'avantage d'être compatible avec toutes les souches industrielles. En effet, toutes les souches industrielles ayant été générées à partir de l'isolat naturel QM6a qui est de type sexuel *MAT1-*2*,* elles sont toutes de type sexuel *MAT1-2,* femelles stériles mais mâles fertiles.

Ladite souche selon l'invention est également femelle fertile, c'est-à-dire qu'elle est capable de différencier des fructifications et d'émettre systématiquement des ascospores (les cellules reproductrices des champignons ascomycètes tels que *T. reesei*) lors d'un croisement avec une souche femelle stérile *MAT1-2.* Ladite souche selon l'invention est également mâle fertile.

Ladite souche selon l'invention présente également l'avantage de ne pas être considérée comme un organisme génétiquement modifié (OGM), et n'est donc pas soumise à une obligation de traçabilité.

Selon un mode de réalisation de l'invention, ladite souche s'entend d'une souche isolée et/ou purifiée.

Dans un deuxième aspect, l'invention concerne aussi l'utilisation d'une souche selon l'invention, c'est-à-dire l'utilisation d'une souche telle que définie ci-dessus.

L'invention concerne ainsi l'utilisation d'une souche telle que définie ci-dessus pour la production d'enzymes cellulolytiques. Selon l'invention, le terme « enzymes cellulolytiques » s'entend plus particulièrement des enzymes cellulases choisies parmi les endoglucanases, les exoglucanases et les glucosidases, et plus particulièrement la β-glucosidase. Le terme « cellulase » fait plus particulièrement référence à une enzyme adaptée à l'hydrolyse de la cellulose et permettant aux microorganismes (tels que *T. reesei*) qui les produisent d'utiliser la cellulose comme source de carbone, en hydrolysant ce polymère en sucres simples (glucose). L'invention concerne donc également l'utilisation d'une souche telle que définie ci-dessus pour l'hydrolyse de la cellulose ou de la lignocellulose en glucose.

L'invention concerne également l'utilisation de la souche telle que définie ci-dessus pour la production de produits biosourcés à partir du glucose. Selon l'invention, le terme « produit biosourcé » s'entend de tout produit qui n'est pas d'origine fossile et qui ne contient pas de produit organique d'origine fossile. Le terme « produit d'origine fossile » signifie tout produit organique issu du pétrole ou du charbon ou des dérivés du pétrole ou des dérivés du charbon. Préférentiellement, selon l'invention, il s'agit d'un alcool, tel que l'isopropanol, le butanol ou l'éthanol, et plus particulièrement l'éthanol. Selon un mode de réalisation, l'invention s'entend également d'un procédé de production de produits biosourcés à partir du glucose comprenant l'utilisation des enzymes cellulolytiques produites par une souche de *Trichoderma reesei* telle que définie ci-dessus. Selon ce mode de réalisation l'invention concerne ainsi une étape de mise en contact entre du glucose et les enzymes cellulolytiques produites par une souche de *Trichoderma reesei* telle que définie ci-dessus.

L'invention concerne également l'utilisation d'une souche telle que définie ci-dessus pour la production de biocarburant. Ainsi, l'invention concerne un procédé de production d'un biocarburant à partir de substrats cellulosiques ou lignocellulosiques, comprenant l'utilisation des enzymes cellulolytiques produites par une souche de *Trichoderma reesei* telle que définie ci-dessus. L'invention s'entend aussi d'un procédé de production d'un biocarburant à partir de substrats cellulosiques ou lignocellulosiques, comprenant :
- une étape de prétraitement d'un substrat cellulosique ou lignocellulosique afin d'obtenir un substrat prétraité,
- une étape d'hydrolyse enzymatique du substrat prétraité, en présence d'un mélange (ou cocktail) d'enzymes produites par une souche de *T. reesei* telle que définie ci-dessus et d'un substrat approprié, afin d'obtenir un hydrolysat,
- une étape de fermentation alcoolique de l'hydrolysat obtenu,
- une étape de distillation.

Les conditions de chacune des étapes (températures, durées, ...) pourront être aisément déterminées par l'homme du métier, de même que le substrat approprié. Par exemple, le lactose ou la cellulose, ou leurs mélanges, sont des substrats classiquement utilisés pour induire la production de cellulases par *T. reesei,* bien que d'autres substrats puissent être envisagés. Selon un mode de réalisation de l'invention, dans le procédé de production d'un biocarburant, la souche de *T. reesei* sert à produire les enzymes cellulolytiques. Selon l'invention, le terme « biocarburant » s'entend de tout produit issu de la transformation de la biomasse et pouvant être utilisé à des fins énergétiques. A titre d'exemple, cela s'entend des biogaz, des produits pouvant être incorporés (éventuellement après transformation ultérieure) à un carburant ou être un carburant à part entière, tels que des alcools (de façon non exclusive l'éthanol, le butanol et/ou l'isopropanol), des solvants (acétone), des acides (butyrique), des lipides et leurs dérivés (acides gras à courtes ou longues chaînes, esters d'acides gras), ainsi que l'hydrogène. De manière préférée, le biocarburant selon l'invention est un alcool, par exemple l'éthanol, le butanol et/ou l'isopropanol. Plus préférentiellement, le biocarburant selon l'invention est l'éthanol. Dans un mode de réalisation particulier, ledit procédé de production d'un biocarburant comprend une étape de récupération du biocarburant produit.

Dans un mode de réalisation particulier, l'invention concerne également un procédé de production de β-glucosidase comprenant l'étape de culture d'une souche de *T. reesei* telle que définie ci-dessus dans un milieu de culture comprenant un substrat approprié, notamment choisi parmi le lactose et la cellulose ou un de leurs mélanges. Dans un mode de réalisation particulier, ledit procédé de production de β-glucosidase peut comprendre une étape de récupération de β-glucosidase.

Dans un troisième aspect, l'invention concerne aussi un procédé de reproduction sexuée entre deux souches de *Trichoderma reesei,* ledit procédé comprenant une étape de croisement entre une première souche telle que définie ci-dessus et une deuxième souche compatible. *T. reesei* est un champignon dit hétérothallique bipolaire, c'est-à-dire que la reproduction sexuée n'est possible qu'entre individus de type sexuel compatibles (*MAT1-1* et *MAT1-2*). Selon l'invention « une deuxième souche compatible » s'entend ainsi d'une souche de *T. reesei* de type sexuel *MAT1-2.* Avantageusement la « deuxième souche compatible » est femelle stérile et mâle fertile. Selon un mode de réalisation préféré, la deuxième souche compatible est choisie parmi les souches NG-14 (ATCC 56767), RutC30 (ATCC 56765), Cl847, QM9414 (ATCC 26921), QM9123 (ATCC 24449), et Tu-6 (ATCC MYA256), de préférence les souches RutC30, Cl847, QM9414 et Tu-6. Ces souches sont bien connues de l'homme du métier. La souche selon l'invention peut être utilisée pour réaliser des croisements avec d'autres souches industrielles issues de mutagénèses -afin de les améliorer-, mais aussi avec des souches issues de manipulation génétique -par exemple afin d'éliminer des marqueurs de sélection ou bien pour combiner des caractéristiques/propriétés intéressantes-. En d'autres termes, le procédé de reproduction sexuée selon l'invention permet avantageusement (i) l'amélioration non OGM de souches industrielles de *T. reesei* par reproduction sexuée, mais également (ii) l'élimination des marqueurs de sélection par croisement de cette souche avec une souche industrielle OGM ou (iii) l'obtention de nouvelles souches aux propriétés/caractéristiques intéressantes. La souche selon l'invention peut ainsi être considérée comme une souche « outil » et peut être croisée avec d'autres souches industrielles afin d'améliorer leurs performances. Dans un aspect, l'invention concerne ainsi l'utilisation d'une souche de *T. reesei* telle que définie ci-dessus pour améliorer les propriétés d'une souche compatible, notamment industrielle. Le terme « souche compatible » s'entend d'une souche de *T. reesei* de type sexuel *MAT1-2,* notamment femelle stérile et mâle fertile. Le terme « souche industrielle » s'entend d'une souche issue de la souche QM6a ; en d'autres termes une souche femelle stérile et mâle fertile. Le terme « améliorer les propriétés d'une souche industrielle » signifie que le croisement entre la souche selon l'invention et une souche compatible, notamment industrielle, permet d'obtenir et/ou de sélectionner une descendance ayant des propriétés améliorées. En d'autres termes, la souche selon l'invention peut être utilisée, pour améliorer par croisement avec une souche compatible, notamment industrielle, les propriétés de la nouvelle souche obtenue. Le terme « propriétés améliorées » peut s'entendre d'une meilleure viscosité, d'une meilleure productivité, d'une meilleure stabilité, ... La viscosité d'une souche peut être déterminée par toutes techniques usuelles pour l'homme du métier, tel que par exemple le protocole décrit par N. Hardy *et al.* La stabilité d'une souche peut également être déterminée par toutes techniques usuelles pour l'homme du métier, tel que par exemple le protocole décrit par N. Hardy *et al.* Selon l'invention, la « stabilité d'une souche » s'entend de la conservation au cours du temps des propriétés telles que la productivité, la constitution du cocktail d'enzymes, la morphologie, ...

L'invention sera mieux illustrée par les exemples et les figures suivantes. Les exemples ci-après visent à éclaircir l'objet de l'invention et illustrer des modes de réalisation avantageux, et en aucun cas vise à restreindre la portée de l'invention.

### FIGURES

La **Figure 1** représente la production de protéines en fioles alimentées.
La **Figure 2** représente le principe du procédé de production de cellulases d'IFP Énergies nouvelles.
La **Figure 3A** représente l'évaluation de la quantité de biomasse au cours de la fermentation dans un bioréacteur DASGIP.
La **Figure 3B** représente l'évaluation de la produite quantité de protéines produites au cours de la fermentation dans un bioréacteur DASGIP.
La **Figure 4** représente la généalogie des souches obtenues à partir de la souche QM6a. Les souches marquées d'une étoile ont été testées en croisement avec la souche RuA149 (voir l'exemple 2). Les références des souches présentées sur la Figure sont les suivantes :
   (1) Mandels, M., Weber, J., & Parizek, R. (1971). Enhanced cellulase production by a mutant of Trichoderma viride. Applied microbiology, 21(1), 152-4.
   (2) Mandels M. (1975).Microbial sources of cellulase. Biotechnol Bioeng.5, 81-105.
   (3) Gruber, F., Visser, J., Kubicek, C. P., & De Graaff, L. H. (1990). The development of a heterologous transformation system for the cellulolytic fungus Trichoderma reesei based on a pyrG-negative mutant strain. Current genetics, 18(1), 71-6.
   (4) Durand, H., Clanet, M., & Tiraby, G. (1988). Genetic improvement of Trichoderma reesei for large scale cellulase production. Enzyme and microbial technology, 10(June), 341-346.
   (5) Eveleigh DE, Montenecourt BS (1979) Increasing yields of extracellular enzymes. Adv Appl Microbiol 25:57-74.
   (6) Montenecourt BS, Eveleigh DE(1977). Preparation of Mutants of Trichoderma reesei with Enhanced Cellulase Production. Applied and environmental microbiology, 34(6), 777-782.

### EXEMPLES

### Exemple 1 : Obtention de la souche selon la présente invention (i.e. la souche telle que déposée sous le numéro CNCM I-5221) et détermination de ses caractéristiques

### 1. Obtention

La souche hyper-productrice de cellulases RutC30 (ou Rut-C30) (Montenecourt and Eveleigh, 1977) a été croisée avec la souche A2 selon le protocole décrit dans Seidl *et al..* La souche RutC30 (déposée à l'ATCC sous le numéro 56765) a été obtenue par trois étapes de mutagénèse depuis la souche QM6a et est une des meilleures productrices de cellulases du domaine public (Peterson and Nevalainen, 2012).

Après le croisement, plusieurs campagnes d'isolement d'ascospores ont été effectuées. 295 descendants purifiés ont été obtenus. Ces derniers ont été ensuite soumis à un criblage en trois étapes. Ces cribles successifs permettent de réduire le nombre de candidats de quelques centaines à l'individu le plus performant pour la production de cellulases.

### 2. Production de cellulases - Test en microplaque

La première étape du criblage repose sur une méthode de mesure de la production de protéines extracellulaires par la méthode Bradford permettant de classer les candidats et visant à en réduire drastiquement le nombre pour ne transférer à la seconde étape qu'une dizaine des individus les plus intéressants.

Le second crible est basé sur une miniaturisation des conditions industrielles (Jourdier *et al.,* 2012) et permet de sélectionner quelques individus pour lesquelles les performances seront testées en bioréacteurs.

Lors du premier crible, les descendants sont cultivés en microplaque dans 2 mL de milieu inducteur de cellulase (tel que Avicel®, commercialisé par MSDS sous la référence No.: 9004-34-6).

Après sept jours de culture, la production de cellulases est mesurée par le dosage de la quantité de protéines totales sécrétées dans le surnageant selon la méthode de Bradford. Une corrélation entre protéines totales sécrétées et cellulases peut être faite car chez *T. reesei,* les exoglucanases (CBHI, CBHII) et les endoglucanases (EGI, EGII) principales peuvent représenter jusqu'à 90% de la quantité totale de protéines sécrétées (Markov et al. 2005). Seuls les dix meilleurs producteurs de cellulases, présentant un écart-type inférieur à 10%, ont été sélectionnés.

### 3. Production de cellulases - Miniaturisation des conditions industrielles

Les dix meilleurs producteurs de cellulases ont été sélectionnés pour être soumis à un second crible qui utilise le protocole « fioles alimentées » développé par Jourdier et al., 2012 et qui se base sur la miniaturisation d'un protocole fed-batch permettant de maximiser la production d'enzymes (Jeude et al., 2006). Ce protocole permet de produire des cellulases en fiole avec une alimentation contrôlée par un mélange stoechiométrique de substrat carboné et de base NH3, ce qui permet de stabiliser le pH de la culture sans système de contrôle du pH. Un incubateur avec un agitateur rotatif est utilisée pour contrôler l'agitation et la température (Jourdier et al., 2012).

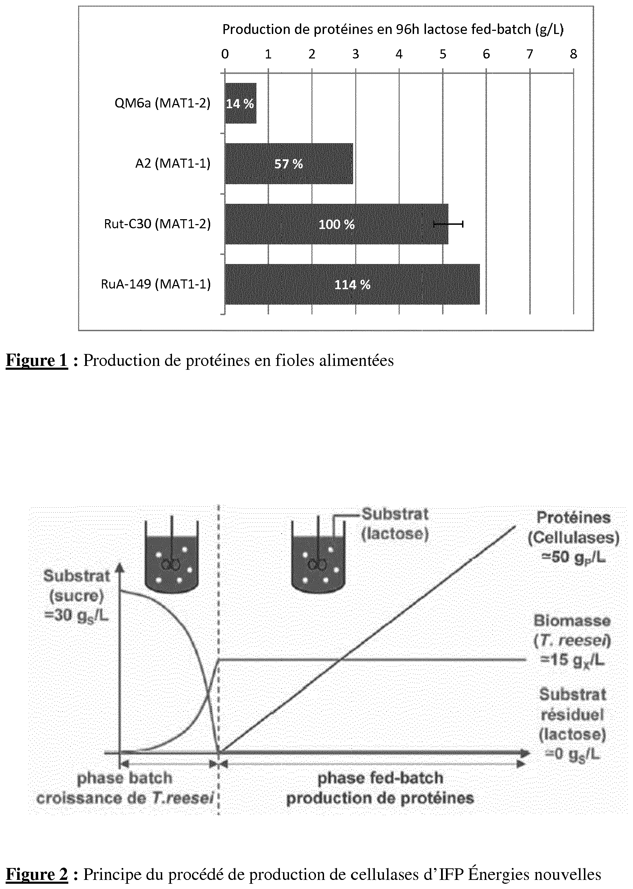

La méthodologie des fioles alimentées permet de tester huit souches en parallèle. Chaque fiole est alimentée par des pompe avec un débit de 0.3 mL/h avec une solution contenant 50 g/L de lactose, 0.8 g/L de (NH4)2SO4, et 160 mM NH3. A 96h, les protéines totales sécrétées sont dosées. Les résultats des souches QM6A, A2, RutC30 et RuA-149 sont présentés en **Figure 1****.** La production de protéines en fioles alimentées n'a été évalué qu'une seule fois pour toutes les souches sauf pour la souche de référence RutC30. Les pourcentages représentent le taux de production de protéines par rapport à la souche de référence RutC30.

Cette méthode de production permet de classer les souches selon leur production de protéines sécrétées qui correspondent chez *T. reesei* à des cellulases (Markov et al., 2005). Après un fed-batch en lactose de 96h, le dosage des protéines produites mesurées selon la méthode de Lowry (**Figure 1**) confirme que la souche A2 produit plus de protéines que la souche QM6a et deux fois moins que la souche hyper-productrice RutC30.

Grace à ce second crible, la souche RuA-149 a été sélectionnée pour être testée en bioréacteurs qui miment à petite échelle les conditions industrielles. La culture en bioréacteur s'effectue selon le protocole de production IFP Énergies nouvelles (Pourquie et al., 1988) et qui distingue deux phases (**Figure 2**) :
- une phase de batch d'environ 30h qui correspond à une phase de croissance de *T*. *reesei* qui produit de la biomasse jusqu'à la consommation totale du glucose ;
- une phase de fed-batch d'environ 200h au cours de laquelle la production de cellulases est induite par un ajout à débit constant de lactose afin de maximiser et de maintenir constante la vitesse la production.

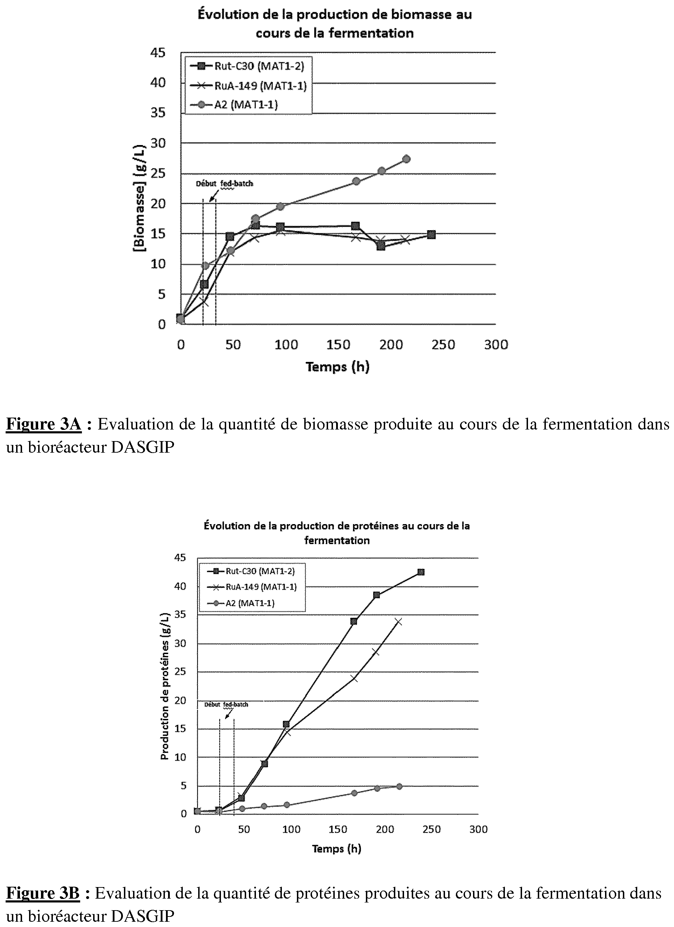

La production de biomasse (**Figure 3A**) et de protéines (**Figure 3B**) (dosages avec la méthode de Lowry) ont été évaluées tout au long de la fermentation dans les bioréacteurs DASGIP. Les activités spécifiques Filter Paper (FP) (activité des endoglucanases et exoglucanases) et β-glucosidase sont dosées uniquement pour les prélèvements finaux (après 200h). Ces expérimentations ont été répétées deux fois et les résultats obtenus sont similaires.

Les résultats sont présentés dans le **Tableau 1** ci-après.

**Tableau 1 : Activités spécifiques Filter Paper (FP) et β-glucosidase des souches parentes (A2, RutC30 et de la descendance RuA-149).**

| **Souche** | **Activité FP (IU/mg)** | **Activité β-glucosidase (IU/mg)** |
|---|---|---|
| **RutC30** | 0,77 ± 0,11 | 0,45 ± 0,07 |
| **A2** | 0,41 ± 0,01 | 0,72 ± 0,03 |
| **RuA-149** | 0,8 ± 0,13 | 1,33 ± 0,09 |

La souche RuA-149 produit progressivement environ 5g/L de biomasse jusqu'à la consommation totale du glucose qui intervient après 34h. Durant la phase de fed-batch, la biomasse se stabilise autour de 15g/L (**Figure 3A**).

La production de protéines par la souche RuA-149 débute aussitôt l'induction faite mais elle est ralentie à partir de 100h. La production de protéines de la souche RuA-149 est de 34g/L (**Figure 3B**).

L'hydrolyse efficace de la cellulose nécessite l'action synergique des endoglucanases, exoglucanases et des β-glucosidase (Kubicek et al., 2009). Les endoglucanases et exoglucanases peuvent représenter jusqu'à 90% de la quantité totale de protéines sécrétées tandis que la β-glucosidase représente moins de 1% (Lynd et al., 2002; Herpoël-Gimbert et al., 2008). La β-glucosidase principale (codée par le gène *bgl1* ou *cel3a* chez *T. reesei*) intervient pour hydrolyser le cellobiose (dimère) lors de la dernière étape avant l'obtention du monomère de glucose (Chauve *et al.,* 2010). Sa faible proportion dans le cocktail cellulolytique de *T. reesei* entraine une accumulation de cellobiose dont l'effet inhibiteur sur les exoglucanases entraine un ralentissement significatif de l'hydrolyse (Chauve et al., 2010). Une augmentation de l'activité β-glucosidase dans le cocktail enzymatique est essentielle pour une hydrolyse plus efficace lors de la production du bioéthanol de deuxième génération. En fin de fermentation, la qualité du cocktail enzymatique produit est évaluée par la mesure des activités enzymatiques (**Figures 3A** et **3B**). L'activité des endoglucanases et exoglucanases est dosée par le test FP (filter-paper) qui donne une indication de l'efficacité globale du cocktail de cellulases (Ghose, 1987). L'activité limitante de la β-glucosidase est évaluée séparément par mesure de l'hydrolyse de p-nitrophenyl-β-Dglucopyranoside (pNPG). Le dosage FP montre que la souche A2 présente une activité cellulolytique spécifique faible par rapport à la souche de référence RutC30 mais son activité β-glucosidase est presque deux fois supérieure à celle de la RutC30. La souche selon l'invention, RuA-149, a une activité FP globale similaire à celle de la souche de référence RutC30 mais elle présente une activité β-glucosidase environ trois fois supérieure.

Ces résultats montrent que le croisement entre la souche A2 et la souche hyper-productrice RutC30 a permis de générer une souche hyper-productrice améliorée pour l'activité β-glucosidase.

### Exemple 2 : Obtention de croisements fertiles entre la souche selon la présente invention (i.e. la souche telle que déposée sous le numéro CNCM I-5221) avec des souches MAT1-2 femelles stériles

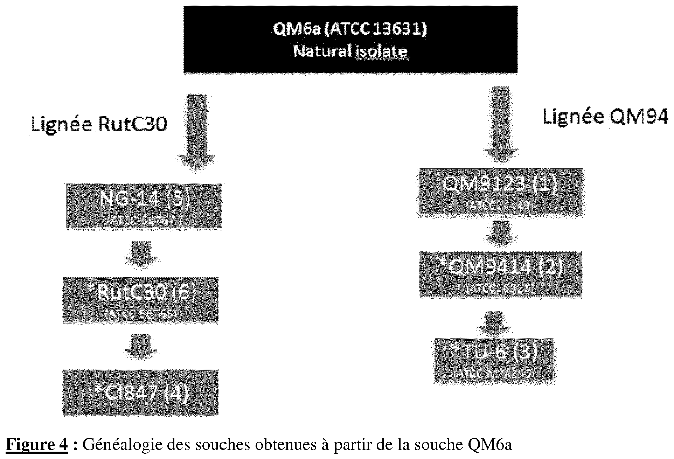

La souche RuA-149 a été croisée avec des souches de *Trichoderma reesei MAT1-2* et femelles stériles issus de l'isolat QM6a. Les souches A2 et QM6a *MAT1-1 IDC1* (Linke *et al.,* 2015) ont été utilisées comme contrôle positif (croisement fertile). Les croisements ont été réalisés par confrontation : les souches sont ensemencées en ligne l'une en face de l'autre, avec environ 1,5 cm entre les deux souches. Chaque ligne contient de quatre à six inocula de la même souche provenant soit d'une boîte de conidies soit d'un cryotube. Un croisement est fertile lorsqu'il y a expulsion des ascospores à partir des périthèces contenus dans les stromata. L'expulsion des ascospores est observée par la présence d'une substance jaunâtre présente sur les ostioles des périthèces. Les croisements ont été effectués avec les souches RutC30, Cl847, QM9414 et Tu-6. Les résultats sont présentés dans le **Tableau 2** ci-après. La généalogie des souches testées est présentée en **Figure 4****.**

**Tableau 2: Croisement entre la souche selon l'invention et des souches MAT1-2 femelles stériles**

| **Souches testées (*MAT1-2* femelles stériles)** | **Croisement avec A2** (contrôle positif) | **Croisement avec la souche RuA-149** |
|---|---|---|
| RutC30 | Fertile | Fertile |
| C1847 | Fertile | Fertile |
| QM9414 | Fertile | Fertile |
| Tu-6 | Fertile | Fertile |

Ces résultent confirment que la souche selon l'invention peut être croisée avec des souches femelles fertiles, et ainsi générer de nouvelles souches.

### Références

Bradford, M. (1976). A rapid and sensitive method for the quantitation of microgram quantities of protein utilizing the principle of protein-dye binding. Analytical Biochemistry 72, 248-254.
Chauve, M., Mathis, H., Huc, D., Casanave, D., Monot, F., and Lopes Ferreira, N. (2010). Comparative kinetic analysis of two fungal beta-glucosidases. Biotechnology for biofuels 3, 3.
Ghose, T.K. (1987). Measurement of cellulase activities. Pure and Applied Chemistry 59. Herpoël-Gimbert, I., Margeot, A., Dolla, A., Jan, G., Mollé, D., Lignon, S., Mathis, H., Sigoillot, J., Monot, F., and Asther, M. (2008). Comparative secretome analyses of two Trichoderma reesei RUT-C30 and CL847 hypersecretory strains. Biotechnology for biofuels 1, 18.
Jeude, M., Dittrich, B., Niederschulte, H., Anderlei, T., Knocke, C., Klee, D., and Büchs, J. (2006). Fed-batch mode in shake flasks by slow-release technique. Biotechnology and bioengineering 95, 433-445.
Jourdier, E., Poughon, L., Larroche, C., Monot, F., and Ben Chaabane, F. (2012). A new stoichiometric miniaturization strategy for screening of industrial microbial strains: application to cellulase hyper-producing Trichoderma reesei strains. Microbial cell factories 11, 70.
Kubicek, C.P., Mikus, M., Schuster, A., Schmoll, M., and Seiboth, B. (2009). Metabolic engineering stratégies for the improvement of cellulase production by Hypocrea jecorina. Biotechnology for biofuels 2, 19.
Hardy N., Moreaud M., Guillaume D., Augier F., Nienow A., Béal C., Ben Chaabane F., (2017). Advanced digital image analysis method dedicated to the characterization of the morphology of filamentous fungus. Journal of Microscopy. 266, 2, 126-140.
Linke R., Thallinger G., Haarmann T., Eidner J., Schreiter M., Lorenz P., Seiboth B., Kubicek C., (2015). Restoration of female fertility in Trichoderma reesei QM6a provides the basis for inbreeding in this industrial cellulase producing fungus. Biotechnol Biofuels. 2015; 8: 155
Lowry OH, Rosebrough NJ, Farr AL, Randall RJ, (1951). Protein measurement with the Folin phenol reagent. J Biol Chem, 193:265-275.
Lynd, L.R., Weimer, P.J., van Zyl, W.H., and Pretorius, I.S. (2002). Microbial Cellulose Utilization. Fundamentals and Biotechnology. Microbiology and molecular biology reviews 66, 506-577.
Markov, A.V., Gusakov, A.V., Kondratyeva, E.G., Okunev, O.N., Bekkarevich, A.O., and Sinitsyn, A.P. (2005). New Effective Method for Analysis of the Component Composition of Enzyme Complexes from Trichoderma reesei. Biochemistry (Moscow) 70, 657-663.
Montenecourt, B.S., and Eveleigh, D.E. (1977). Preparation of mutants of Trichoderma reesei with enhanced cellulase production. Applied and environmental microbiology 34*.*
Peterson, R., and Nevalainen, H. (2012). Trichoderma reesei RUT-C30--thirty years of strain improvement. Microbiology 158, 58-68.
Pourquie, J., Warzywoda, M., Chevron, F., Thery, D., and Lonchamp, D. (1988). Scale up of cellulase production and utilization. In FEMS Symposium n°43: Biochemistry and Genetics of Cellulose Dégradation., J.-P. Aubert, P. Beguin and J. Millet, eds., pp. 71-86.
Seidl, V., Seibel, C., Kubicek, C.P., and Schmoll, M. (2009). Sexual development in the industrial workhorse Trichoderma reesei. Proceedings of the National Academy of Sciences of the United States of America 106, 13909-13914.

## Revendications

1. Souche de *Trichoderma reesei* telle qu'enregistrée le 03 août 2017 à la CNCM sous le numéro CNCM I-5221.

2. Utilisation d'une souche selon la revendication 1 pour la production d'enzymes cellulolytiques, notamment les cellulases, et plus particulièrement la β-glucosidase.

3. Utilisation d'une souche selon la revendication 1 pour l'hydrolyse de la cellulose ou de la lignocellulose en glucose.

4. Utilisation d'une souche selon la revendication 1 pour la production de produits biosourcés à partir du glucose.

5. Procédé de production de produits biosourcés à partir du glucose comprenant l'utilisation des enzymes cellulolytiques produites par une souche de *Trichoderma reesei* selon la revendication 1.

6. Utilisation d'une souche selon la revendication 1 pour la production de biocarburant.

7. Procédé de production d'un biocarburant à partir de substrats cellulosiques ou lignocellulosiques, comprenant l'utilisation des enzymes cellulolytiques produites par une souche de *Trichoderma reesei* selon la revendication 1.

8. Procédé de production d'un biocarburant à partir de substrats cellulosiques ou lignocellulosiques selon la revendication 7, comprenant :
- une étape de prétraitement d'un substrat cellulosique ou lignocellulosique afin d'obtenir un substrat prétraité,
- une étape d'hydrolyse enzymatique du substrat prétraité, en présence d'un mélange d'enzymes produites par une souche de *Trichoderma reesei* selon la revendication 1 et d'un substrat approprié, afin d'obtenir un hydrolysat,
- une étape de fermentation alcoolique de l'hydrolysat obtenu,
- une étape de distillation.

9. Procédé de production de β-glucosidase comprenant l'étape de culture d'une souche de *Trichoderma reesei* selon la revendication 1 dans un milieu de culture comprenant un substrat approprié.

10. Procédé de reproduction sexuée entre deux souches de *Trichoderma reesei,* ledit procédé comprenant une étape de croisement entre une première souche selon la revendication 1 et une deuxième souche compatible.

11. Procédé de reproduction sexuée entre deux souches de *Trichoderma reesei* selon la revendication 10, dans laquelle la deuxième souche compatible est choisie parmi les souches NG-14, RutC30, Cl847, QM9414, QM9123, Tu-6, de préférence les souches RutC30, Cl847, QM9414 et Tu-6.

12. Utilisation d'une souche de *T. reesei* selon la revendication 1 pour améliorer les propriétés d'une souche compatible, notamment industrielle.
